(19) **Europäisches Patentamt**

**European Patent Office**

**Office européen des brevets**

(11) **EP 0 550 293 B1**

(12) **FASCICULE DE BREVET EUROPEEN**

(45) Date de publication et mention
de la délivrance du brevet:
**17.05.2000 Bulletin 2000/20**

(51) Int. Cl.⁷: **A61N 1/365**

(21) Numéro de dépôt: **92402762.6**

(22) Date de dépôt: **09.10.1992**

(54) **Stimulateur cardiaque à fréquence asservié à l'effort du patient**

Herzschrittmacher mit einer an die Belastung eines Patienten angepassten Reizungsfrequenz

Cardiac pacemaker with a stimulation frequency controlled by the patient's effort

(84) Etats contractants désignés:
**BE CH DE ES FR GB IT LI SE**

(30) Priorité: **31.12.1991 FR 9116368**

(43) Date de publication de la demande:
**07.07.1993 Bulletin 1993/27**

(73) Titulaire:
**ELA MEDICAL (Société anonyme)**
**F-92541 Montrouge Cédex (FR)**

(72) Inventeurs:
• **Legay, Thierry**
**F-91640 Fontenay les Briis (FR)**

• **Ripart, Alain**
**F-91190 Gif-sur-Yvette (FR)**

(74) Mandataire: **Laget, Jean-Loup**
**Cabinet Loyer,**
**78, avenue Raymond Poincaré**
**75116 Paris (FR)**

(56) Documents cités:
**EP-A- 0 080 348**      **EP-A- 0 149 572**
**EP-A- 0 414 928**      **WO-A-90/08570**
**DE-A- 2 838 854**      **US-A- 4 771 780**
**US-A- 5 012 316**

## Description

L'invention concerne un stimulateur cardiaque à fréquence asservie à l'effort du patient.

Pour apprécier l'effort du patient, il est possible de mesurer l'accélération selon un axe, par exemple vertical, ou antéro-postérieur. Un accéléromètre placé sur le tronc du patient permet d'obtenir une bonne approximation de la dépense énergétique pour les efforts dynamiques. Il existe une bonne corrélation entre l'intégrale de la valeur absolue du signal d'accélération selon l'axe vertical, par exemple, et la dépense énergétique du patient. Le calcul de cette intégrale sur des périodes de temps fixes donne une image de la puissance fournie par le patient dans ses efforts dynamiques, qui peut être choisie comme paramètre d'asservissement de la fréquence cardiaque.

Le document US-4.926.863 décrit un stimulateur cardiaque à fréquence asservie à la mesure de l'accélération selon un axe antéro-postérieur.

Le document Us 4 771 780 décrit un capteur de mouvement basé sur le mouvement d'une bille à l'intérieur d'une enceinte close munie de contacts électriques.

Le document EP A 0 080 348 décrit un capteur piézoélectrique solidaire du boîtier de façon à enregistrer les vibrations transmises audit boîtier.

Le document US A 5 012 316 décrit un capteur multiaxial en une seule pièce et son interconnexion.

Un but de l'invention est de proposer un stimulateur cardiaque possédant un algorithme d'asservissement de la fréquence de stimulation à l'effort du patient, le le paramètre d'asservissement étant représentatif de la puissance métabolique.

Un autre but de l'invention est de proposer un stimulateur de ce type, fournissant une information toujours cohérente, même si le boîtier du stimulateur tourne après implantation, et un paramètre d'asservissement particulièrement fiable.

L'invention a pour objet un stimulateur cardiaque à fréquence asservie à l'effort du patient, sur un paramètre calculé à partir de la mesure de l'accélération au niveau du tronc du patient, comportant un boîtier présentant une face plane, et deux accéléromètres disposés à l'intérieur du boîtier, sensibles chacun dans la direction d'un axe, les deux axes étant perpendiculaires entre eux et parallèles à la face plane du boîtier du stimulateur, caractérisé en ce que le paramètre d'asservissement est une fonction des valeurs des signaux représentatifs de l'accélération suivant lesdits axes, en relation avec la puissance métabolique dépensée par le patient au cours de son effort, et reste sensiblement invariant en cas de rotation du boîtier.

Selon d'autres caractéristiques de l'invention :

- le stimulateur comporte un accéléromètre sensible selon un troisième axe perpendiculaire aux deux précédents. ;
- le paramètre d'asservissement est l'intégrale, calculée sur un temps déterminé, de la somme des valeurs absolues des signaux représentatifs de l'accélération suivant lesdits axes;
- le paramètre d'asservissement est l'intégrale, calculée sur un temps déterminé, de la racine carrée de la somme des carrés des signaux représentatifs de l'accélération suivant lesdits axes ;
- l'accéléromètre est à variation de capacité ;
- l'accéléromètre est constitué avec un substrat plat, l'accéléromètre étant sensible dans une direction contenue dans le plan du substrat ;
- deux accéléromètres sont montés sur le même substrat et orientés de façon à être sensibles dans deux directions perpendiculaires ;
- un troisième accéléromètre est prévu, sensible dans une direction perpendiculaire au plan dudit substrat ;
- le troisième accéléromètre est monté dans le plan dudit substrat ;
- le troisième accéléromètre est monté sur ledit substrat ;
- le stimulateur comporte un ensemble intégrateur à grande constante de temps dont le signal de sortie est délivré sous forme numérique ;
- l'ensemble intégrateur comporte un intégrateur qui génère une rampe rapide avec une constante de temps de quelques millisecondes, un comparateur à tension de référence, un monostable délivrant une impulsion à chaque basculement du comparateur, et un compteur délivrant sous forme numérique un signal de sortie ;
- un micro-processeur détermine, à partir du signal numérique de sortie du compteur, la fréquence cardiaque asservie.

D'autres caractéristiques ressortent de la description qui suit faite avec référence au dessin annexé sur lequel on peut voir :

Figure 1 - Un schéma symbolique de la chaîne électronique de mesure de la puissance métabolique à partir de capteurs d'accélération selon trois axes orthogonaux, et d'élaboration des impulsions de stimulation à fréquence asservie, le paramètre d'asservissement étant l'intégrale de la somme des valeurs absolues des signaux représentatifs de l'accélération suivant lesdits axes.

Figure 2 - Un schéma analogue à celui de la Fig. 1 dans lequel le paramètre d'asservissement est l'intégrale de la racine carrée de la somme des carrés desdits signaux.

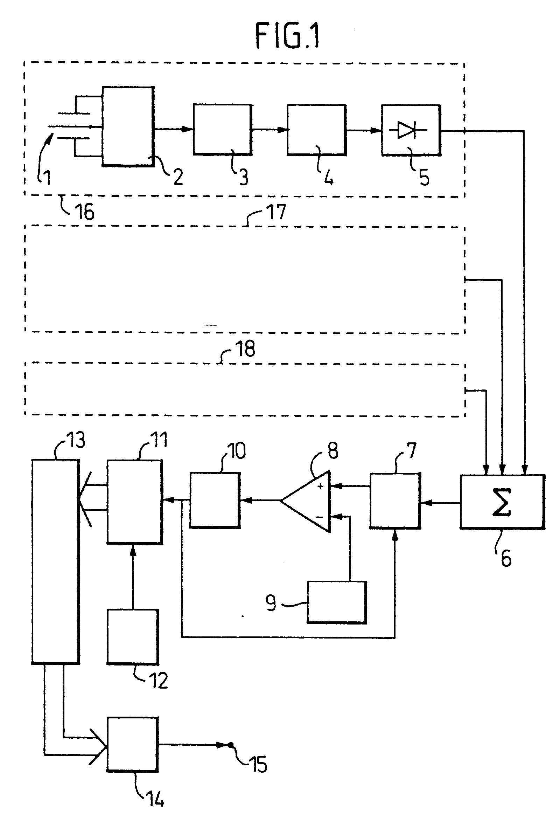

Sur la figure 1, trois axes orthogonaux sont prévus pour la mesure de l'accélération : un axe horizontal antéro-postérieur, un axe vertical et un axe latéral. Pour chacun de ces axes, une série de modules électroniques est prévue, respectivement 16, 17, 18. Chaque série comporte cinq modules :

- un capteur accélérométrique 1, de type capacitif, à variation de capacité, micro-usiné sur substrat de silicium par exemple, et présentant un axe sensible ;
- un amplificateur 2 donnant une variation de tension pour une variation de capacité dans le capteur 1 ; sa sortie donne typiquement 270 mV/g, où g est l'accélération de la pesanteur;
- un filtre passe-bas 3 pour éliminer les composantes parasites du signal d'accélération recueilli par le capteur ; sa fréquence de coupure est de quelques Hz, typiquement 6 Hz ;
- un filtre passe-haut 4, à fréquence de coupure inférieure à 1 Hz, de préférence égale à 0,7 Hz par exemple, pour éliminer la composante statique du champ gravifique terrestre ;
- et un redresseur double-alternance 5 donnant la valeur absolue du signal d'accélération.

Pour chacun des trois axes, la valeur absolue du signal d'accélération est appliquée à un sommateur 6. Un intégrateur 7 reçoit le résultat de la sommation par le sommateur 6 et génère une rampe rapide, avec une constante de temps de quelques ms, typiquement 3,9ms. La sortie de l'intégrateur 7 est appliquée à une entrée d'un comparateur 8, dont l'entrée de référence reçoit une tension de référence en provenance du circuit 9 générateur de tension de référence réglable.

La sortie du comparateur 8 est reliée à un monostable 10 qui fournit une impulsion à chaque basculement du comparateur 8. La sortie du monostable 10 est reliée à l'entrée de remise à zéro de l'intégrateur 7 et à l'entrée d'un compteur 11.

L'ensemble des modules 7 à 11 constitue un intégrateur à grande constante de temps dont le signal de sortie est directement obtenu sous forme numérique. Le fonctionnement en est le suivant :

L'intégrateur 7 génère une rampe rapide en un temps relativement court. Lorsque la sortie de l'intégrateur dépasse la tension de référence en provenance du circuit 9, le comparateur 8 bascule, déclenchant le monostable 10 qui fournit une impulsion au compteur 11. Cette impulsion incrémente le compteur 11 et remet à zéro l'intégrateur 7. le processus recommence alors de façon identique.

L'ajustage de la tension de référence permet de régler le gain de la chaîne électronique pour adapter la chaîne à des capteurs de sensibilités différentes.

L'horloge 12 définit le temps de calcul de la puissance métabolique, par exemple 1,5 s : toutes les 1,5s, le résultat du compteur 11 est mémorisé dans des bascules, puis ce compteur est remis à zéro pour un nouveau cycle de mesure.

Le micro-processeur 13 acquiert la mesure de la puissance métabolique Pmet et réalise le calcul de la fréquence cardiaque Fc par une relation linéaire :

$$Fc = A.Pmet + Fc.base$$

Le paramètre Fc.base correspondant à la fréquence cardiaque de base, est fixé par le médecin. La pente A de l'asservissement est fixée par le médecin, mais elle peut être recalculée automatiquement pour s'ajuster au mieux au patient.

Pour la détermination de la fréquence de stimulation cardiaque, la fréquence Fc calculée n'est prise en compte que si elle diffère de la fréquence de base de plus de 6%.

Le circuit 14 est un circuit intégré spécifique. Il reçoit du microprocesseur 13 les caractéristiques de largeur, d'amplitude et de rythme, des impulsions de stimulation à appliquer et il génère ces impulsions qui sont appliquées à l'électrode 15 d'une sonde de stimulation.

Le paramètre d'asservissement est fonction de la puissance métabolique Pmet dépensée par le patient lors de l'effort. Le calcul de cette puissance est réalisé en évaluant la dépense énergétique du patient sur une période de temps fixe égale à 1,5s dans l'exemple décrit. Cette période est fixée par l'horloge 12. La dépense énergétique est obtenue en intégrant la somme des valeurs absolues des signaux d'accélération mesurés suivant les axes des capteurs accélérométriques.

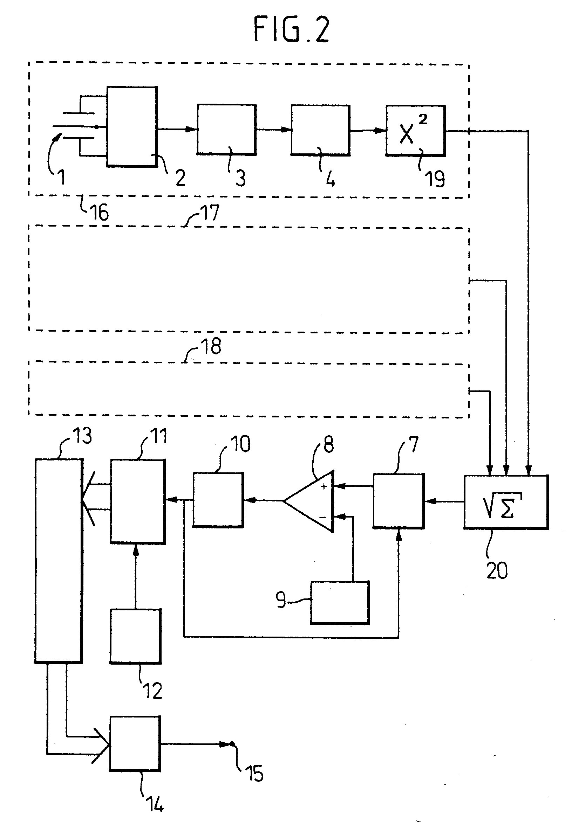

Sur la figure 2, les mêmes éléments que sur la Fig. 1 sont désignés par les mêmes références. Les modifications par rapport à la Fig. 1 sont les suivantes :

- le redresseur double-alternance 5 donnant la valeur absolue du signal d'accélération selon un axe, est remplacé par un quadrateur 19 donnant le carré du signal d'accélérations selon cet axe.
  Chacune des séries de modules électroniques 16, 17, 18 donne le carré du signal d'accélération selon son axe.
- le sommateur 6 donnant la somme des vapeurs absolues des signaux selon chacun des trois axes, est remplacé par un module électronique 20 donnant la racine carrée de la somme des carrés des signaux d'accélération selon chacun des trois axes.

Dans le cas de la Fig. 2, la mesure de l'accélération est mathématiquement précise. Dans le cas de la Fig. 1, cette mesure est approchée, et en cas de rotation du boîtier, il peut en résulter une erreur de mesure pouvant aller jusqu'à 25% environ, compensée par un algorithme de calibration automatique de la relation entre Fc et Pmet, par exemple.

Selon l'invention, les capteurs accélérométriques se présentent sous forme de puces, disposées sur le circuit électronique du stimulateur cardiaque, à l'intérieur du boîtier du stimulateur. L'ensemble des capteurs constitue un accéléromètre multidimensionnel pour améliorer l'estimation de la puissance fournie par le patient dans son effort dynamique.

Un exemple de capteur accélérométrique susceptible d'être utilisé dans le cadre de l'invention est décrit dans le document EP 0 149 572.

Dans un substrat, en silicium par exemple, est prévu un évidement traversant le substrat et définissant dans le substrat une tige flexible, orientée suivant une direction parallèle à la surface du substrat, et susceptible de se déformer suivant une direction perpendiculaire à sa direction d'orientation, et parallèle à la surface du substrat. Cette tige est donc capable de mesurer, par variation capacité, la composante de l'accélération selon ladite direction perpendiculaire à sa direction d'orientation.

Avec deux capteurs de ce type sensibles dans les directions perpendiculaires X et Y d'un plan, et un capteur sensible dans la direction Z perpendiculaire à ce plan, les trois capteurs étant disposés dans ledit plan, ou avec des capteurs équivalents pour mesurer les accélérations dans trois directions perpendiculaires, les composantes de l'accélération sont toutes connues.

Les avantages de cette disposition sont notamment :

- une information complète sur les mouvements du tronc, et en conséquence, une meilleur approximation de la dépense énergétique par unité de temps, c'est-à-dire un paramètre d'asservissement plus fiable ;
- une insensibilité à la position du boîtier et à la position du patient, car le repère de mesure est orthonormé;
- un bon rapport signal/bruit.

Les accéléromètres à variation de capacité, présentent en outre les caractéristiques suivantes :

- une excellente stabilité dans le temps,
- une très faible dérive en température,
- une très grande sensibilité,
- un encombrement minimal car leur masse active est très petite.

Un capteur accélérométrique du type décrit est sensiblement plat ; la direction d'orientation de la tige et la direction de détection des accélérations sont parallèles à la surface du substrat et perpendiculaires entre elles. Sur un même substrat plat, on peut disposer deux capteurs sensibles dans deux directions perpendiculaires. Le troisième capteur doit être disposé dans le même plan mais être sensible selon une direction perpendiculaire à ce plan. L'accéléromètre selon l'invention est constitué sous forme de puce.

Il arrive que l'utilisation de deux capteurs soit estimée suffisante. Dans ce cas, les deux capteurs détectent de préférence les accélérations selon l'axe vertical et l'axe latéral (séries de modules électroniques 17 et 18 de la Fig. 1). L'information fournie est sensiblement constante même si le boîtier du stimulateur tourne après implantation, avec les mêmes restrictions que précédemment relatives au traitement des signaux.

Le cas de deux capteurs est considéré comme entrant dans le cadre de l'invention, comme defini dans la revendication 1.

**Revendications**

1. Stimulateur cardiaque à fréquence asservie à l'effort du patient, sur un paramètre calculé à partir de la mesure de l'accélération au niveau du tronc du patient, comportant un boîtier présentant une face plane, et deux accéléromètres disposés à l'intérieur du boîtier, sensibles chacun dans la direction d'un axe, les deux axes étant perpendiculaires entre eux et parallèles à la face plane du boîtier du stimulateur, caractérisé en ce que le paramètre d'asservissement est une fonction des valeurs des signaux représentatifs de l'accélération suivant lesdits axes, en relation avec la puissance métabolique dépensée par le patient au cours de son effort et reste sensiblement invariant en cas de rotation du boîtier.

2. Stimulateur selon la revendication 1, caractérisé en ce qu'il comporte un troisième accéléromètre sensible selon un troisième axe perpendiculaire aux deux précédents.

3. Stimulateur selon l'une des revendications 1 ou 2, caractérisé en ce que le paramètre d'asservissement est l'intégrale, calculée sur un temps déterminé, de la somme des valeurs absolues des signaux représentatifs de l'accélération suivant lesdits axes.

4. Stimulateur selon l'une des revendications 1 ou 2, caractérisé en ce que le paramètre d'asservissement est l'intégrale, calculée sur un temps déterminé, de la racine carrée de la somme des carrés des signaux représentatifs de l'accélération suivant lesdits axes.

5. Stimulateur selon l'une des revendications 1 ou 2, caractérisé en ce que ledits accéléromètres (1) sont a variation de capacité.

6. Stimulateur selon l'une des revendications 1 ou 2, caractérisé en ce que ledits accéléromètres sont constitués avec des substrats plats, chaque accéléromètre étant sensible dans une direction contenue dans le plan de son substrat.

7. Stimulateur selon la revendication 6, caractérisé en ce que deux accéléromètres sont montés sur le même substrat et orientés de façon à être sensibles dans deux desdites directions perpendiculaires.

**8.** Stimulateur selon la revendication 7, caractérisé en ce que: un troisième accéléromètre est prévu, sensible dans une direction perpendiculaire au plan dudit substrat.

**9.** Stimulateur selon la revendication 8, caractérisé en ce que le troisième accéléromètre est monté dans le plan dudit substrat.

**10.** Stimulateur selon la revendication 8, caractérisé en ce que le troisième accéléromètre est monté sur ledit substrat.

**11.** Stimulateur selon la revendication 3, caractérisé en ce qu'il comporte un ensemble intégrateur (7-11) à grande constante de temps dont le signal de sortie est délivré sous forme numérique.

**12.** Stimulateur selon la revendication 11, caractérisé en ce que l'ensemble intégrateur (7-11) comporte un intégrateur (7) qui génère une rampe rapide avec une constante de temps de quelques millisecondes, un comparateur (8) à tension de référence, un monostable (10) délivrant une impulsion à chaque basculement du comparateur (8), et un compteur (11) délivrant sous forme numérique un signal de sortie.

**13.** Stimulateur selon la revendication 12, caractérisé en ce que un micro-processeur (13) détermine, à partir du signal numérique de sortie du compteur (11), la fréquence cardiaque asservie.

**Claims**

**1.** A pacemaker with frequency controlled by the patient's effort according to a parameter computed from the acceleration measured at the level of the patient's trunk, comprising a box having a flat surface, and two accelerometers placed inside the box, each of which being sensitive along the direction of one axes, the two axes being perpendicular to one another and parallel to the flat surface of the pacemaker box, characterised in that the control parameter is a function of the values representing the acceleration along said axes, in relation to the metabolic power used up by the patient in the course of the effort and remains essentially invariant in case of rotation of the box.

**2.** The pacemaker as claimed in claim 1, characterised in that it comprises a third accelerometer being sensitive along a third axes perpendicular to the two previous axes.

**3.** The pacemaker as claimed in claim 1 or 2, characterised in that the control parameter is the integral, computed for a predetermined period of time, of the sum of the absolute values of said signals representing the acceleration along said axes.

**4.** The pacemaker as claimed in claim 1 or 2, characterised in that the control parameter is the integral, computed for a predetermined period of time, of the square root of the sum of the squares of said signals representing the acceleration along said axes.

**5.** The pacemaker as claimed in claim 1 or 2, characterised in that the accelerometers (1) have capacity variation.

**6.** The pacemaker as claimed in claim 1 or 2, characterised in that said accelerometers are comprised of a flat substrata and that each accelerometer is sensitive in one direction contained in the plane of its substratum.

**7.** The pacemaker as claimed in claim 6, characterised in that two of said accelerometers are mounted on the same substratum and oriented so as to be sensitive in two of said perpendicular directions.

**8.** The pacemaker as claimed in claim 7, characterised in that said third accelerometer is provided, and is sensitive in a direction perpendicular to the plane of said substratum.

**9.** The pacemaker as claimed in claim 8, characterised in that said third accelerometer is mounted in the plane of said substratum.

**10.** The pacemaker as claimed in claim 8, characterised in that said third accelerometer is mounted on said substratum.

**11.** The pacemaker as claimed in claim 3, comprising an integrating unit (7-11) that is highly constant in time and of which the output signal is supplied in digital form.

**12.** The pacemaker as claimed in claim 11, characterised in that said integrating unit (7-11) comprises an integrator (7) which generates a fast ramp with a time constant of a few milliseconds, a reference voltage comparator (8), a monostable circuit (10) issuing a pulse at each flip of said comparator (8), and a counter (11) issuing an output signal in digital form.

**13.** The pacemaker as claimed in claim 12, characterised in that a microprocessor determines, out of the digital output signal from said counter (11), the controlled heart rate.

**Patentansprüche**

1. Herzschrittmacher mit von der Belastung des Patienten abhängiger, über einen beruhend auf der Messung der Beschleunigung auf dem Niveau des Rumpfes des Patienten berechneten Parameter geregelter Frequenz, mit

   einem eine ebene Vorderseite aufweisenden Gehäuse, und
   zwei im Inneren des Gehäuses angeordneten Beschleunigungsmessern, von denen jeder in der Richtung einer Achse empfindlich ist, wobei die zwei Achsen zueinander senkrecht und parallel zu der ebenen Vorderseite des Gehäuses des Herzschrittmachers sind, **dadurch gekennzeichnet, dass**
   der Regelungsparameter eine Funktion der Werte der die Beschleunigung entlang der Achsen wiedergebenden Signale, bezogen auf die von dem Patienten im Verlauf seiner Belastung verbrauchte Stoffwechselenergie ist und im Fall der Drehung des Gehäuses im wesentlichen unverändert bleibt.

2. Herzschrittmacher nach Anspruch 1, **gekennzeichnet durch** einen dritten Beschleunigungsmesser, der entlang einer dritten Achse empfindlich ist, die zu den beiden vorherigen Achsen senkrecht ist.

3. Herzschrittmacher nach einem der Ansprüche 1 oder 2,
   **dadurch gekennzeichnet, dass**
   der Regelungsparameter das über eine bestimmte Zeit berechnete Integral der Summe der Absolutwerte der die Beschleunigung entlang der Achsen wiedergebenden Signale ist.

4. Herzschrittmacher nach einem der Ansprüche 1 oder 2,
   **dadurch gekennzeichnet, dass**
   der Regelungsparameter das über eine bestimmte Zeit berechnete Integral der Quadratwurzel der Summe der Quadrate der die Beschleunigung entlang der Achsen wiedergebenden Signale ist.

5. Herzschrittmacher nach einem der Ansprüche 1 oder 2,
   **dadurch gekennzeichnet, dass**
   die Beschleunigungsmesser (1) von einer auf einer Kapazitätsveränderung beruhenden Bauart sind.

6. Herzschrittmacher nach einem der Ansprüche 1 oder 2,
   **dadurch gekennzeichnet, dass**
   die Beschleunigungsmesser mit ebenen Substraten gebildet sind, wobei jeder Beschleunigungsmesser in einer in der Ebene seines Substrats enthaltenen Richtung empfindlich ist.

7. Herzschrittmacher nach Anspruch 6,
   **dadurch gekennzeichnet, dass**
   zwei Beschelunigungsmesser auf dem selben Substrat angebracht sind und derart ausgerichtet sind, dass sie in zwei der senkrechten Richtungen empfindlich sind.

8. Herzschrittmacher nach Anspruch 7,
   **dadurch gekennzeichnet, dass**
   ein dritter Beschleunigungsmesser vorgesehen ist, der in einer zu der Ebene des Substrats senkrechten Richtung empfindlich ist.

9. Herzschrittmacher nach Anspruch 8,
   **dadurch gekennzeichnet, dass**
   der dritte Beschleunigungsmesser in der Ebene des Substrats angebracht ist.

10. Herzschrittmacher nach Anspruch 8,
    **dadurch gekennzeichnet, dass**
    der dritte Beschleunigungsmesser auf dem Substrat angebracht ist.

11. Herzschrittmacher nach Anspruch 3,
    **dadurch gekennzeichnet, dass**
    er eine Integratoranordnung (7 - 11) mit grosser Zeitkonstante aufweist, deren Ausgangssignal in numerischer Form ausgegeben wird.

12. Herzschrittmacher nach Anspruch 11,
    **dadurch gekennzeichnet, dass**
    die Integratoranordnung (7 - 11)

    einen Integrierer (7), der eine schnelle Rampe mit einer Zeitkonstanten von einigen Millisekunden erzeugt,
    einen Vergleicher (8) mit einer Bezugsspannung,
    ein monostabiles Kippglied (10), das einen Impuls bei jedem Kippen des Vergleichers (8) abgibt, und
    einen ein Ausgangssignal in numerischer Form ausgebenden Zähler (11) aufweist.

13. Herzschrittmacher nach Anspruch 12,
    **dadurch gekennzeichnet, dass**
    ein Mikroprozessor (13) beruhend auf dem numerischen Ausgangssignal des Zählers (11) die geregelte Herzfrequenz bestimmt.

# FIG.1

# FIG.2